# EUROPEAN PATENT SPECIFICATION

(11) **EP 4 038 198 B1**
(45) Date of publication and mention of the grant of the patent: **06.12.2023**
(21) Application number: 20789689.5
(22) Date of filing: 02.10.2020
(51) Int. Cl.: C12Q 1/6855, C12Q 1/6834

(54) **METHOD**
VERFAHREN
PROCÉDÉ

(30) Priority: 03.10.2019 GB 201914282
(43) Date of publication of application: 10.08.2022
(73) Proprietor: Stemnovate Limited, Cambridge CB23 6DP (GB)
(72) Inventor: SHARMA, Ruchi, Cambridge CB23 6DP (GB); AKANDE, Femi, Cambridge CB23 6DP (GB)
(74) Representative: Mishcon de Reya LLP
(86) International application number: PCT/IB2020/059298
(87) International publication number: WO 2021/064697

(56) References cited:
- WO-A1-2011/150168
- WO-A1-2018/134616
- WO-A2-2012/078312

## Description

### Field of the Invention

The present invention relates to methods and apparatus for synthesising polynucleotides such as DNA and RNA in the absence of a template, to polynucleotides synthesised therefrom and to a kit of parts for synthesising polynucleotides.

### Background to the Invention

Since the early 1980s, synthetic polynucleotides have been manufactured through a series of chemical processes using building blocks called nucleoside phosphoramidites. These can be normal or modified nucleosides which have protecting groups to prevent their amines, hydroxyl groups and phosphate groups from interacting incorrectly. One phosphoramidite is added at a time, the 5' hydroxyl group is deprotected and a new base is added and so on. The chain grows in the 3' to 5' direction, the reverse of what happens in nature. At the end of the synthesis, all protecting groups are removed.

The core chemical phosphoramidite technology is somewhat limited in terms of purity. The longer the oligonucleotide sequence being synthesised, the more defects there are. Thus, the process is only practical for producing short sequences of nucleotides. The current practical limit is about 200 bp (base pairs). Additionally, the process is slow, having long coupling times. Furthermore, the current methodology is heavily reliant on harsh, non-environmentally friendly chemicals and generates toxic byproducts e.g. acetonitrile, toluene and trichloroacetic acid.

WO2011150168, WO2012078312 and WO2018134616 each disclose synthesis of polynucleotides by iterative adapter ligation steps where a glycosylase cleavage at the penultimate nucleotide allows for all but one defined base to be left in the growing synthesis strand.

Thus, there is an unmet need to be able to produce polynucleotides of longer length (for example >150bp) and of high purity. It is further desired to reduce the environmental impact of polynucleotide synthesis and to provide a process with shorter coupling times (i.e. a quicker process).

It is the aim of the presently disclosed invention to provide an alternative method of polynucleotide synthesis that will significantly improve the limitations of the current chemical synthesis methods.

### Summary of the Invention

According to a first aspect of the invention, there is provided a method of synthesising single or double-stranded polynucleotide comprising the steps:
a) providing a polynucleotide starter strand comprising, in sequence from 5' to 3', a nucleotide strand selected from the group consisting:
   (i) 5' blocker - 5'end - segment 1- CUCM 1- segment 2 - 3' end wherein the 3' end is either immobilised or blocked, and,
      optionally, a hybridised complementary strand comprising, in sequence from 5' to 3' 5' end - segment 2C - CUCM2 - segment 1C - 3' end - 3' blocker wherein the 5' end is either immobilised or blocked,
   (ii) 5' blocker-5' end-segment 1 - CUCM1 - segment 2- HP1 - segment 2C-CUCM2-segment 1C - 3'end - 3' blocker wherein HP1 is optionally immobilised, and
   (iii) segment 1 - CUCM1 - segment 2 - HP1 - segment 2C - CUCM2 - segment 1C - HP2 wherein segment 1 is linked to HP 2 to form a double hairpin structure, wherein either HP1 or HP2 may optionally have a HP fluorophore attached and wherein, optionally, the other of HP1 and HP2 is immobilised,
b) digesting CUCM1 with glycosylase enzyme 1 or CUCM2 with a glycosylase enzyme 2 and washing the starter strand to remove the remnant of the digest,
c) where the method is employed to synthesise double stranded polynucleotide, digesting the other of CUCM1 with a first glycosylase enzyme 1 or CUCM2 with glycosylase enzyme 2 and washing to remove the remnant of the digest,
d) ligating a first ligation strand comprising: 5' blocker - 5' end - segment 3 - CUCM3 - additional nucleotide 1-3' end, and/or a second ligation strand comprising: 5' end - additional nucleotide 2 - CUCM4 - segment 3C - 3' end - 3' blocker to the starter strand, and
e) repeating steps b) to d) as many times as required to provide the desired synthetic polynucleotide.

According to a second aspect there is provided a synthetic polynucleotide produced by the method of the disclosure.

According to a third aspect there is provided a kit of parts comprising:
a) a polynucleotide starter strand comprising, in sequence from 5' to 3', a nucleotide strand selected from the group consisting:
   (i) 5' blocker - 5' end - segment 1- CUCM 1 - segment 2-3' end wherein the 3' end is either immobilised or blocked and, optionally, a hybridised complementary strand comprising, in sequence from 5' to 3' 5' end - segment 2C - CUCM2 - segment 1C - 3' end - 3' blocker wherein the 5' end is either immobilised or blocked,
   (ii) 5' blocker-5'end -segment 1 - CUCM1- segment 2 - HP 1-segment 2C-CUCM2 - segment 1C - 3'end - 3' blocker wherein HP1 is optionally immobilised, and
   (iii) segment 1 - CUCM1 - segment 2 - HP 1 - segment 2C - CUCM2 - segment 1C - HP 2 wherein segment 1 is linked to HP 2 to form a double hairpin structure, wherein either HP1 or HP2 optionally has a HP fluorophore attached and wherein, optionally, the other of HP1 and HP2 is immobilised,
   (iv) optionally one or more glycosylase enzymes,
   (v) optionally a ligase enzyme, and
a first ligation strand comprising: 5' blocker - 5'end - segment 1 - CUCM1 - additional nucleotide 1- 3' end, and/or a second ligation strand comprising: 5'end - additional nucleotide 2 - segment 2C - CUCM2 - segment 1C - 3' end - 3' blocker, optionally wherein the first ligation strand and the second ligation strand are joined by a hairpin loop.

Advantageously, this enzymatic DNA/RNA method of template- free polynucleotide synthesis involves the use of highly efficient enzyme to substrate directed interaction in a controlled manner.

### Brief Description of the Drawings

For a better understanding of the invention and to show how the same may be carried into effect, there will now be described by way of example only, specific embodiments, methods and processes according to the present invention with reference to the accompanying drawings in which:

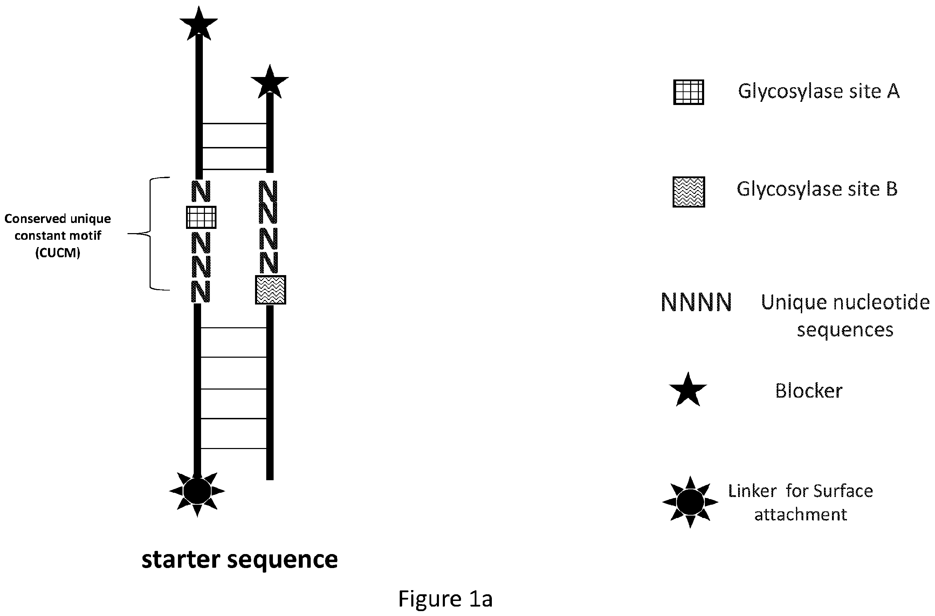

Figure 1a shows an example of a starter sequence for the double stranded, immobilised DNA synthesis method. The example employs glycosylase enzymes, the cutting site for which is shown as a patterned box.

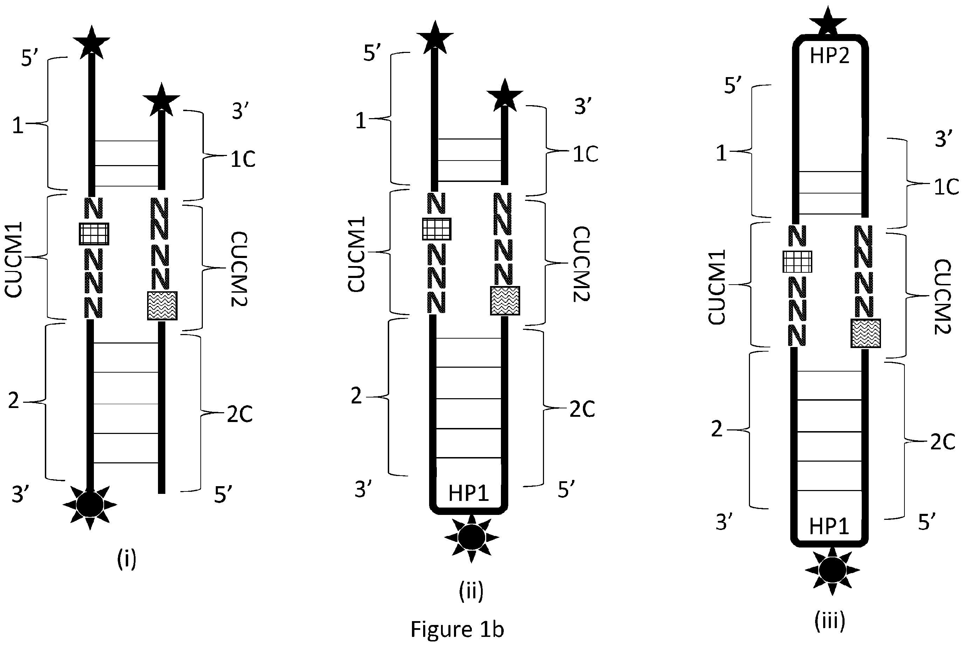

Figure 1b shows a schematic of three variants of double stranded starter strand. (i) shows a starter strand without hairpin, this variant can be employed for single stranded polynucleotide synthesis by omitting one of the complementary strands. (ii) shows a single hairpin loop immobilised. (iii) shows a double hairpin loop with HP1 immobilised. Segments 1, 2, 1C and 2C are shown, as are CUCM1 and CUCM2

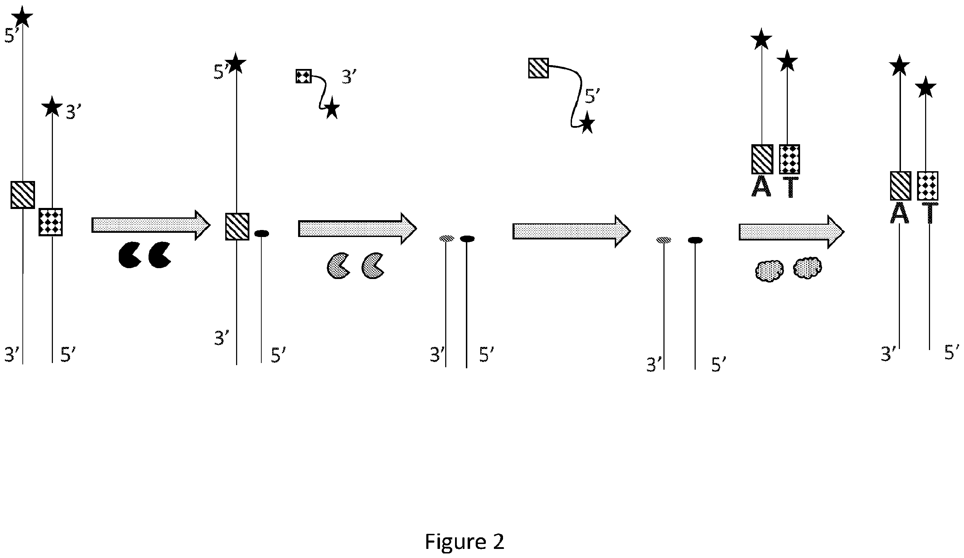

Figure 2 shows an example of the steps of one cycle of an example method in which double stranded nucleotide is synthesised. In this and subsequent figures the patterned boxes are used to denote the entire CUCM for ease of identification. As shown herein, the "pacman" type symbols denote cutting enzymes (e.g. glycosylase) and the "brain" type symbols denote ligase enzymes. For simplification purposes, the nucleotide strands are not shown as immobilised.

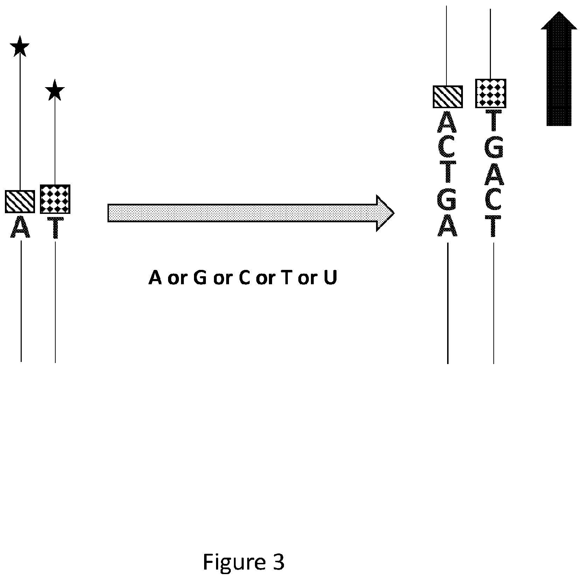

Figure 3 shows an overview of how multiple cycles of the method would grow a synthetic nucleotide. Double stranded nucleotide is shown as an example.
Figures 4 to 7 show the results of a single double stranded, immobilised cycle of the method, wherein:

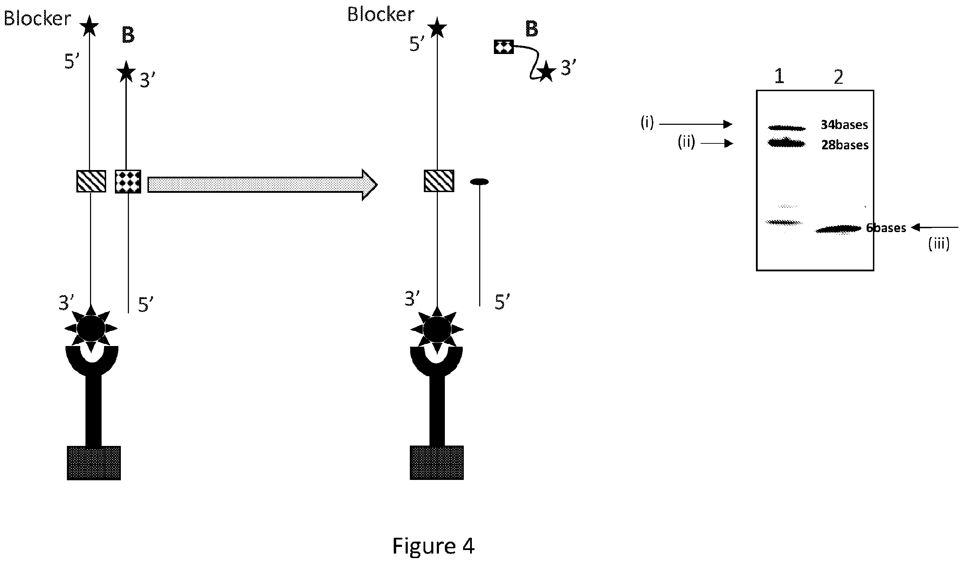

   Figure 4 shows the first cutting step (equivalent to step b)) of the method. Shown is a gel showing lines at (i) 34 and (ii) 28 bases (strand A and strand B respectively) and the (iii) 6 base fragment (digest remnant) washed off strand B. >95% digestion of strand B in lane 2 (at 37°C).

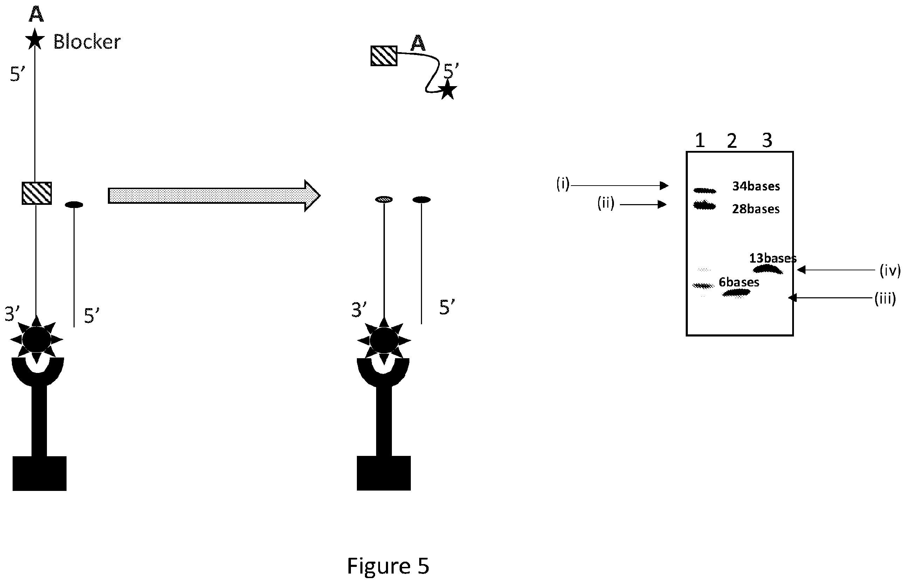

   Figure 5 shows the second cutting step (equivalent to step c)) of the method. Shown in lane 3 is the (iv) 13 base digest remnant washed from strand A.

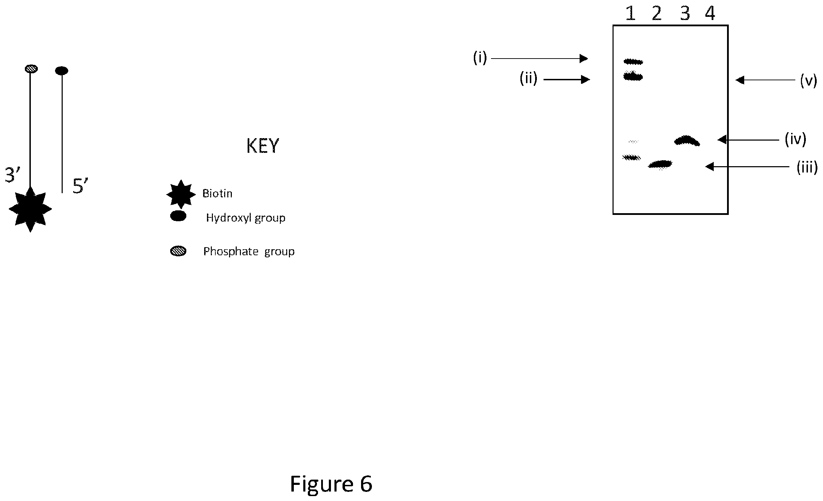

   Figure 6 shows that, once washed, there are no fluorophores in lane 4 (v), indicating that all of the digest remnants have been removed. The remaining strands are shown with blunt ends with their functional groups exposed.

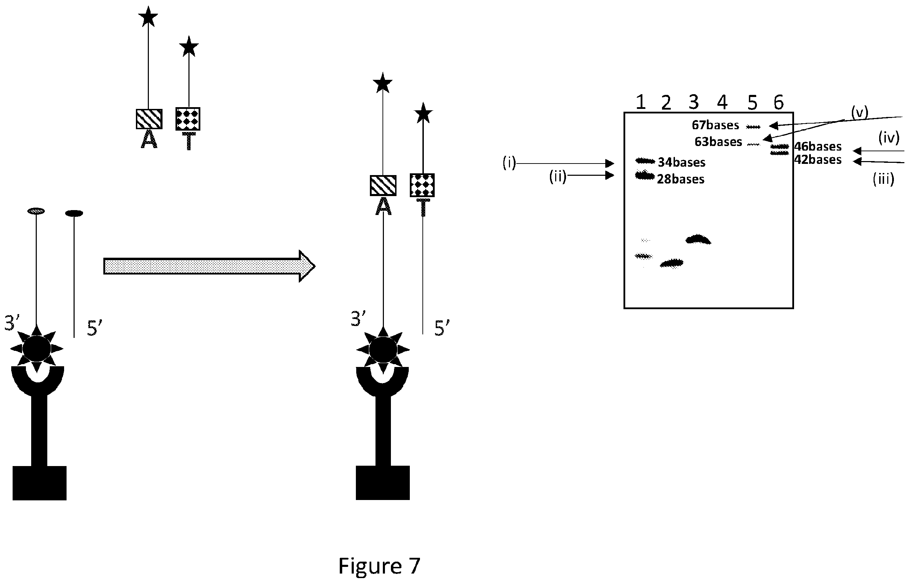

   Figure 7 shows the ligation step wherein the first and second ligation strands are ligated onto the exposed blunt ends. The ligated strands are shown in lane 5 (v) and the first and second ligation strands shown in lane 6 ((iii) and (iv)).

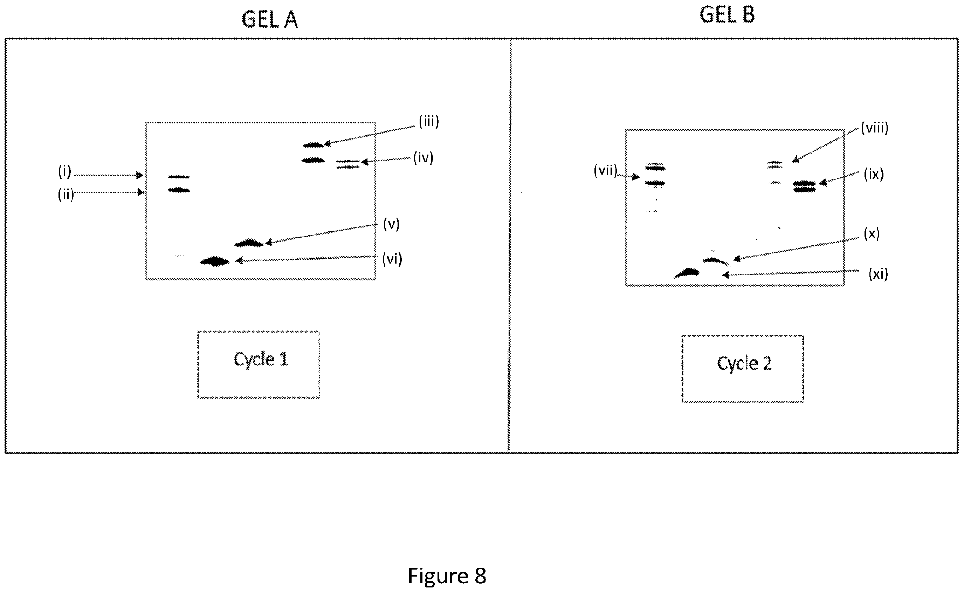

   Figure 8 shows the results after two cycles - i.e. where a two nucleotide strand has been synthesised. (i) indicates strand A, (ii) indicates complimentary strand B, (iii) indicates the ligated new strand (N+1), (iv) indicates control strands, (v) indicates the washed off, digested remnant of strand A and (vi) indicates the washed off, digested remnant of strand B. In cycle 2, a further nucleotide is added to the synthesised strand. (vii) indicates ligated new strands (N+1) used as starting material for the second cycle, (viii) indicates ligated new strand (N+2), (ix) indicates control strands, (x) indicates the washed off, digest remnant of strand A and (xi) indicates the washed off, digested remnant of strand B.

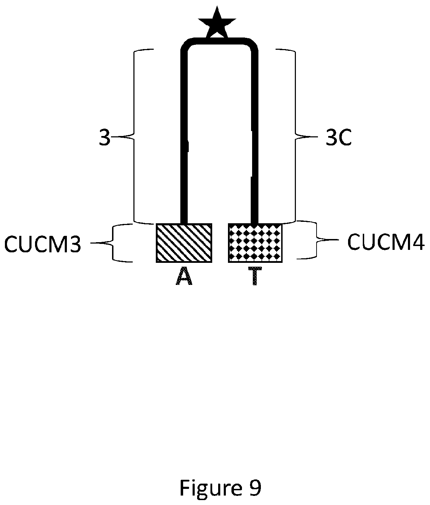

   Figure 9 shows a ligation strand for double stranded synthesis wherein the first ligation strand and the second ligation strand are linked together via a hairpin loop (HP3). Segment 3 and 3C are shown, as are CUCM3 and CUCM4. The figure illustrates the addition of AT nucleotides by way of example only. It will be appreciated that the method is not limited to these two nucleotides.

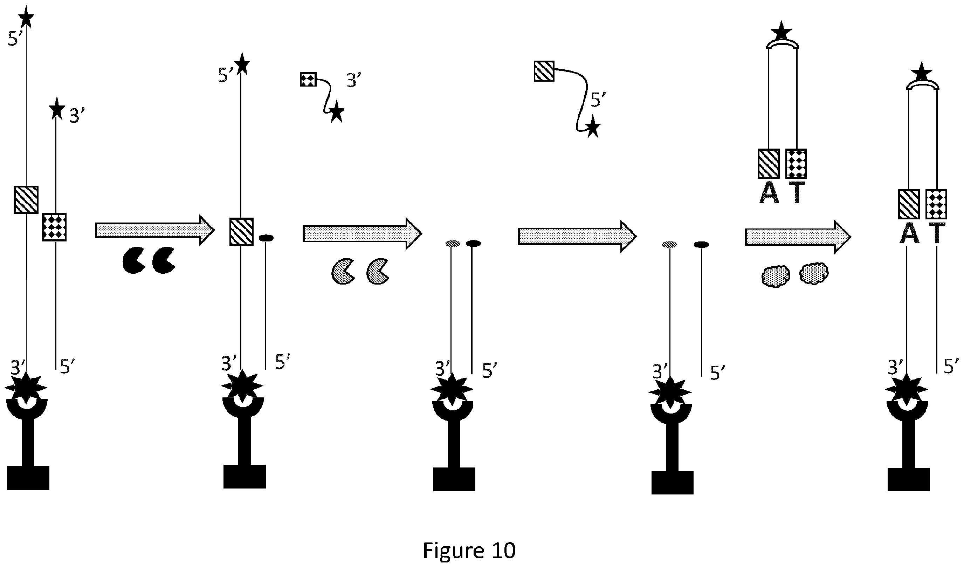

   Figure 10 shows an example of the steps of one cycle of an example method in which double stranded nucleotide is synthesised. This method utilises hairpin linked ligation strands. As shown herein, the "pacman" type symbols denote cutting enzymes (e.g. glycosylase) and the "brain" type symbols denote ligase enzymes. The nucleotide strands are shown as immobilised.

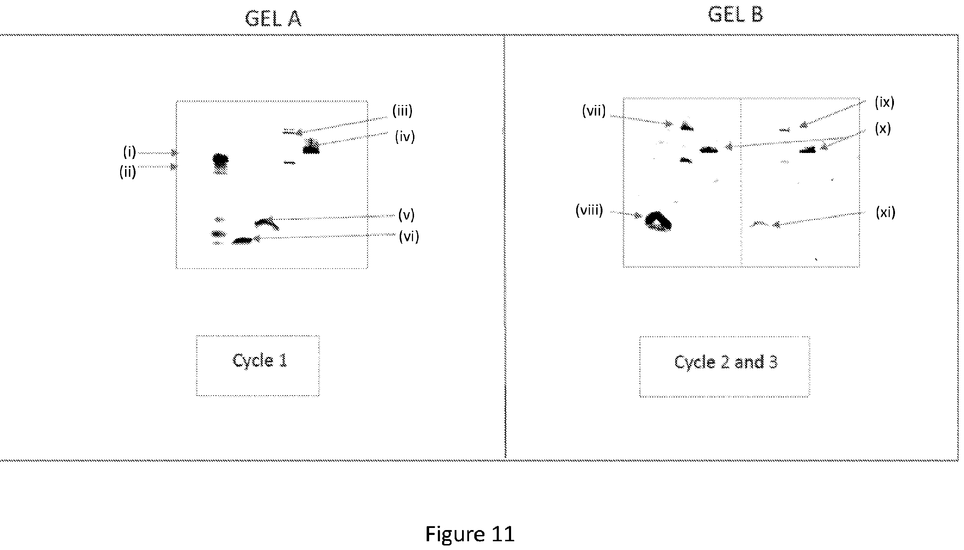

   Figure 11 shows the result of running multiple cycles of the method. (i) indicates starting strand A, (ii) indicates complimentary starting strand B, (iii) indicates the ligated new strand (N+1), (iv) indicates control strands, (v) indicates the washed off, digested remnant of strand A and (vi) indicates the washed off, digested remnant of strand B. Gel B indicates the result following cycle 2 and 3, a further 2 nucleotides are added to the synthesised strand. (vii) indicates ligated new strands (N+2) used as starting material for the third cycle, (viii) indicates washed off, digested remnant of looped, hairpin ligation strand, (ix) indicates ligated new strand (N+3), (x) indicates control strands and (xi) indicates the washed off, digested remnant of looped, hairpin ligation strand.

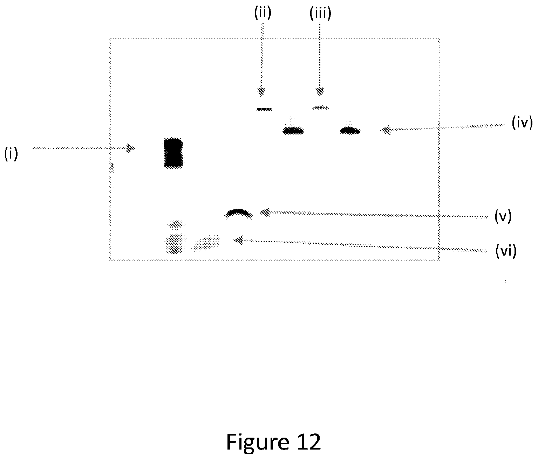

   Figure 12 shows the incorporation of different nucleotides (A, T, C, G) (i) indicates the starting strands, (ii) indicates ligated new nucleotide pair AT, (iii) indicates ligated new nucleotide pair GC, (iv) indicates control strands, (v) indicates the washed off, digest remnant of strand A and (vi) indicates the washed off, digested remnant of strand B

### Detailed Description

As employed herein "synthesising single or double-stranded polynucleotide" refers a method of generating both DNA and RNA. The polynucleotide synthesised may contain entirely standard nucleotides (i.e. CGTAU) or may contain a number of non-standard nucleotides.

As employed herein "starter strand" refers to the polynucleotides, either single or double stranded, used to initiate the method of the invention.

In one embodiment the starter strand is single stranded and comprises the sequence: 5' blocker - 5' end - segment 1 - CUCM1 - segment 2-3' end or 3' blocker - 3' end - segment 2C - CUCM2 - segment 1C - 5' end. Typically, the end that does not have a blocker will be either blocked or immobilised.

In one embodiment the starter strand is double stranded and comprises the sequence: 5' blocker - 5' end - segment 1 - CUCM1 - segment 2-3' end wherein the 3' end is typically either immobilised or blocked and further comprises a hybridised complementary strand comprising the sequence: 5'end - segment 2C - CUCM2 - segment 1C - 3'end - 3' blocker wherein the 5' end is typically either blocked or immobilised. Either strand of the double stranded polynucleotide starter strand can be immobilised, or both can. Where a strand is not immobilised, it is typically blocked.

In one embodiment the double stranded starter strand is a hairpin polynucleotide. That is, the polynucleotide has a stem-loop structure where intramolecular base pairing occurs due to two regions of complementary in-nucleotide sequence (when read in opposite directions) on the same strand of nucleotide. These base-pairs form a double helix that ends in an unpaired loop region.

In one embodiment the hairpin starter strand comprises the sequence: 5' blocker - 5'end - segment 1 -CUCM1-segment 2 - HP 1-segment 2C-CUCM2-segment 1C-3'end - 3' blocker wherein HP1 is optionally immobilised.

In one embodiment the hairpin starter strand is a closed (circular) polynucleotide with complementary sections and loop or hairpin sections at both ends. This structure may comprise a fluorophore attached to one loop/hairpin section and may, independently, be immobilised at the other loop/hairpin section.

In one embodiment the hairpin starter strand comprises the sequence: segment 1 - CUCM1 - segment 2 - HP1 - segment 2C - CUCM2 - segment 1C - HP2 wherein segment 1 is linked to HP2 to form a double hairpin structure. In one embodiment either HP1 or HP2 has a fluorophore attached. In one embodiment the starter strand is immobilised at the HP1 or HP2.

As employed herein blocker refers to a physical or chemical blocker that prevents off-target reactivity. Blockers are designed to inhibit the reactivity of the exposed end (3' or 5') of the nucleotide strands and prevent off-target reactions. Suitable blockers or blocking agents may include, but are not limited to, spacer arms such as spacer C3, spacer C6, spacer C9, spacer C12, spacer C18. Blockers are typically used at the exposed ends of non-immobilised strands in the disclosed method.

In some embodiments the blocker may be a marker for tracking progress of the method, for example, a fluorophore or chromophore. In some embodiments fluorophores may act as blockers.

As employed herein fluorophore is intended to encompass fluorophores and chromophores. Suitable fluorophores include, but are not limited to, TAMRA, Cy3, Cy5, rhodamine red-X, rhodol green, Texas red-X, Oregon green 488/500/514, VIC, Alexa Fluor 488/532/542/555/594/647/750 or FAM. In some embodiments the 5' blocker may be a 5' fluorophore. In some embodiments the 3' blocker may be a 3' fluorophore.

In one embodiment the fluorophore is FAM.

In one embodiment 5' blocker and the 3' blocker are each independently different and selected from the group consisting: a spacer and a fluorophore.

As employed herein 5' and 3' refer to the end of the nucleotide strand as commonly accepted in the art.

As employed herein segment 1 refers to the oligonucleotide (oligo) at the 5' end of the starter strand and located adjacent to and prior to the CUCM1 (in a 5' to 3' direction). Typically, segment 1 is an oligo of at least 20 nucleotides. For example, at least 21, 22, 23, 24, 25, 26, 27, 28, 29, 30, 31, 32, 33, 34, 35 or more nucleotides. Typically, segment 1 is at least 27 nucleotides long. In general, segment 1 is GC rich, that is, it has a high percentage of guanine and cytosine bases (nucleotides), such as over 50% GC content, for example 51, 52, 53, 54, 55, 56, 57, 58, 59, 60, 61, 62, 63, 64, 65, 66, 67, 69, 70, 71, 72, 73, 74, 75, 76, 77, 78, 79, 80, 81, 82, 83, 84, 85% or more. Generally, segment 1 has 55-70% GC content. In one embodiment segment 1 has approximately 60-65% GC content. Advantageously, GC rich oligos hybridise more quickly and efficiently.

As employed herein CUCM refers to a conserved unique constant motif. That is, the region that is conserved between cycles of the method and contains the optimised site of enzymatic function for the digestion steps of the method. CUCMs comprise a distinct nucleotide sequence and a glycosylase recognition site.

In one embodiment the CUCM is 2 to 10 nucleotides long, for example 3, 4, 5, 6, 7, 8 or 9 nucleotides long. Generally, the CUCM is 4 to 8 nucleotides long. In some embodiments the CUCM is 4 nucleotides long.

In one embodiment the glycosylase recognition site is selected from the group consisting of: Uracil, Inosine, 8-OG 8-oxoguanine, 6-MeA 6-methyladenine and 5-hmU 5-hydroxymethyluracil.

In one embodiment the glycosylase recognition site is an Inosine nucleoside. In one embodiment the glycosylase recognition site is a uracil nucleotide.

Typically, CUCM1 and CUCM2 are different but complementary. Typically, CUCM1 and CUCM2 contain different glycosylase recognition sites. In some embodiments it is possible to digest both strands of the polynucleotide at the CUCM sites (CUCM1 and CUCM2) simultaneously or at least within the same digestion step. That is, by providing the same cleavage site in CUCM 1 and CUCM2 or by providing the enzymes to cleave the site at the same time.

Typically, CUCM1 and CUCM2 are complementary to each other where the method is applied to synthesis of double stranded polynucleotides. Where the method is applied to single stranded polynucleotide synthesis, the terms CUCM1 and CUCM2 are used to differentiate between the orientation of the strand relative to blocking, immobilisation, fluorophore sites. It will be appreciated that there is no requirement for the CUCM to be complementary where there is only a single strand. In this context the terminology and syntax of CUCM is for convenience reasons only. Typically, where the 3' end is immobilised, CUCM1 will be used. Where the 5' end is immobilised, CUCM2 will be used.

In one embodiment CUCM1 has the general sequence N(x)ZN(y) wherein N is a nucleotide, x and y are each independently a number in the range 0 to 8 and Z is a glycosylase recognition site. Typically, x and y are each independently is a number selected from 0, 1, 2, 3, 4, 5, 6, 7 or 8. Typically, the sum of x+y is less than 8. In certain embodiments the sum of x+y is 4.

In one embodiment CUCM2 has the general sequence N(x)ZN(y) wherein N is a nucleotide, x and y are each independently a number in the range 0 to 8 and Z is a glycosylase recognition site. Typically, x and y are each independently is a number selected from 0, 1, 2, 3, 4, 5, 6, 7 or 8. Typically, the sum of x+y is less than 8. In certain embodiments the sum of x+y is 4.

As employed herein segment 2 refers to the oligo at the 3' end of the starter strand and located adjacent to and following the CUCM1 (in a 5' to 3' direction). Typically, segment 2 is an oligo of at least 20 nucleotides. For example, at least 21, 22, 23, 24, 25, 26, 27, 28, 29, 30, 31, 32, 33, 34, 35 or more nucleotides. Typically, segment 2 is at least 27 nucleotides long. In general, segment 2 is GC rich, that is, it has a high percentage of guanine and cytosine bases (nucleotides), such as over 50% GC content, for example 51, 52, 53, 54, 55, 56, 57, 58, 59, 60, 61, 62, 63, 64, 65, 66, 67, 69, 70, 71, 72, 73, 74, 75, 76, 77, 78, 79, 80, 81, 82, 83, 84, 85% or more. Generally, segment 2 has 55-70% GC content. In one embodiment segment 2 has approximately 60-65% GC content.

In one embodiment segment 1 and segment 1C are each 20-80 nucleotides long. For example, approximately 25, 30, 35, 40, 45, 50, 55, 60, 65, 70 or 75 nucleotides long.

In one embodiment segment 1 and segment 1C are each at least 25 nucleotides long.

In one embodiment segment 1 and segment 1C are each at most 80 nucleotides long.

In one embodiment segment 1 and segment 1C are each approximately 40 nucleotides long.

In one embodiment segment 2 and segment 2C are each 20-80 nucleotides long. For example, approximately 25, 30, 35, 40, 45, 50, 55, 60, 65, 70 or 75 nucleotides long.

In one embodiment segment 2 and segment 2C are each at least 25 nucleotides long.

In one embodiment segment 2 and segment 2C are each at most 80 nucleotides long.

In one embodiment segment 2 and segment 2C are each approximately 40 nucleotides long.

As employed herein blocked refers to a physical or chemical block designed to inhibit the reactivity of the exposed end (3' or 5') of the nucleotide strands and prevent off-target reactions.

As employed herein immobilised refers to attachment of the starter strand to a physical substrate/surface. The substrate or surface may be any suitable inert surface, for example, glass or beads, for example, functionalised nanowells, microbeads, dynabeads, surface modified borosilicate glass, metal alloy consisting of or comprising Au, Fe, Pd, Co, Zn and Ti.

Immobilisation may take any suitable form, for example, biotin/streptavidin, thiol/ Au bonding, internal amino modifier/ Sulfo SANPAH bonding.

In one embodiment the starter strand is immobilised.

In one embodiment the starter strand is immobilised on streptavidin dynabeads.

As employed herein segment 2C refers to the oligonucleotide (oligo) at the 5' end of the starter strand and located adjacent to and prior to the CUCM2 (in a 5' to 3' direction). Where the method is applied to synthesis of double stranded polynucleotides, segment 2 and segment 2C are typically substantially complementary to each other. Typically, segment 2C is an oligo of at least 20 nucleotides. For example, at least 21, 22, 23, 24, 25, 26, 27, 28, 29, 30, 31, 32, 33, 34, 35 or more nucleotides. Typically, segment 2C is at least 27 nucleotides long. In general, segment 2C is GC rich, that is, it has a high percentage of guanine and cytosine bases (nucleotides), such as over 50% GC content, for example 51, 52, 53, 54, 55, 56, 57, 58, 59, 60, 61, 62, 63, 64, 65, 66, 67, 69, 70, 71, 72, 73, 74, 75, 76, 77, 78, 79, 80, 81, 82, 83, 84, 85% or more. Generally, segment 2C has 55-70% GC content. In one embodiment segment 2C has approximately 60-65% GC content.

As employed herein segment 1C refers to the oligo at the 3' end of the starter strand, located adjacent to and following the CUCM2 (in a 5' to 3' direction). Where the method is applied to synthesis of double stranded polynucleotides, segment 1 and segment 1C are typically substantially complementary to each other. Typically, segment 1C is an oligo of at least 20 nucleotides. For example, at least 21, 22, 23, 24, 25, 26, 27, 28, 29, 30, 31, 32, 33, 34, 35 or more nucleotides. Typically, segment 1C is at least 27 nucleotides long. In general, segment 1C is GC rich, that is, it has a high percentage of guanine and cytosine bases (nucleotides), such as over 50% GC content, for example 51, 52, 53, 54, 55, 56, 57, 58, 59, 60, 61, 62, 63, 64, 65, 66, 67, 69, 70, 71, 72, 73, 74, 75, 76, 77, 78, 79, 80, 81, 82, 83, 84, 85% or more. Generally, segment 1C has 55-70% GC content. In one embodiment segment 1C has approximately 60-65% GC content.

As employed herein HP1 refers to the loop (non-base-paired or single stranded) region of nucleotides in a hairpin starter strand located between segment 2 and segment 2C.

As employed herein HP2 refers to the loop (non-base-paired or single stranded) region of nucleotides in a double hairpin starter strand located between segment 1 and segment 1C. In the double hairpin, the functionalities of HP1 and HP2 are interchangeable. That is, either HP1 or HP2 could be used to immobilise the structure, where immobilised, and likewise either HP1 or HP2 could be used to attach the blocker.

As employed herein digesting CUCM (1/2) refers to the step of enzymatically cleaving the starter strand at a specific site within the CUCM to expose the reactive group on which to grow the nucleotide chain to be synthesised (the 3' or 5' end). Typically, the enzyme used to cleave the starter strand is a glycosylase.

As employed herein glycosylase enzyme 1 is a glycosylase specific to CUCM1, similarly glycosylase enzyme 2 is a glycosylase enzyme specific to CUCM2. In some embodiments the enzymes may be the same although, in general, two different glycosylases will be employed. Glycosylases are enzymes that hydrolyse glycosyl groups. Thus, it follows that that the CUCM(s) will typically contain a glycosyl group.

In one embodiment the product of step c) of the disclosed method is a blunt ended double stranded nucleotide. That is, the exposed free ends-the result of digesting CUCM1 and CUCM2 are the same length, there is no overhang or "sticky end".

In one embodiment steps b) and c) are performed simultaneously.

"washing the construct to remove the remnant of the digest" refers to the step of removing the free oligonucleotide from the reaction mixture following the digestion step. Where the method is carried out in solution, this is achieved by use of a column which retains the strands of interest (i.e. those upon which the nucleotide is being synthesised - or a given length) allowing smaller fragments i.e. cleaved remnants, to pass through the column.

Ligation strand as employed herein refers to one or more oligos which may or may not be substantially identical to the digest remnants. For example, a first ligation strand comprises 5' blocker - 5'end - segment 3 - CUCM3 - additional nucleotide 1- 3' end wherein segment 3 may be identical, similar or different to segment 1 of the starter strand. Typically, segment 3 is identical to segment 1. Similarly and independently, CUCM3 may be identical, similar or different to CUCM1. Typically, CUCM3 is identical to CUCM1. Additional nucleotide 1 is the nucleotide that is added to the synthetic polynucleotide chain being grown in the method. On the first iteration of the method, this will be the first nucleotide, on subsequent iterations of the method it will be added to the growing synthetic polynucleotide chain.

Alternatively, or additionally, ligation strand 2, or the second ligation strand may be employed. Ligation strand 2 comprises 5'end - additional nucleotide 2 - CUCM4 - segment 3C - 3'end - 3' blocker wherein segment 3C may be identical, similar or different to segment 1C of the starter strand. Typically, segment 3C is identical to segment 1C. Similarly and independently, CUCM4 may be identical, similar or different to CUCM2. Typically, CUCM4 is identical to CUCM2. Additional nucleotide 2 is the nucleotide that is added to the synthetic polynucleotide chain being grown in the method. On the first iteration of the method, this will be the first nucleotide, on subsequent iterations of the method it will be added to the growing synthetic polynucleotide chain.

In some embodiments, where double stranded polynucleotide is to be synthesised, the first and second ligation strands are joined via a hairpin loop, for convenience sake referred to as HP3. In this embodiment the ligation strand comprises 5'end - additional nucleotide 2 - CUCM4 - segment 3C - HP3 - segment 3 - CUCM3 - additional nucleotide 1- 3' end.

In some embodiments HP3 has a fluorophore attached.

Typically, segment 3 and segment 3C are complementary to each other and equivalent to segment 1 and segment 1C respectively. That is, where the method is applied to synthesis of double stranded polynucleotides, segment 3 and segment 3C are typically substantially complementary to each other. Typically, segment 3 and segment 3C are oligos of at least 20 nucleotides. For example, at least 21, 22, 23, 24, 25, 26, 27, 28, 29, 30, 31, 32, 33, 34, 35 or more nucleotides. Typically, segment 3 and segment 3C are at least 27 nucleotides long. In general, segment 3 and segment 3C are GC rich, that is, it have a high percentage of guanine and cytosine bases (nucleotides), such as over 50% GC content, for example 51, 52, 53, 54, 55, 56, 57, 58, 59, 60, 61, 62, 63, 64, 65, 66, 67, 69, 70, 71, 72, 73, 74, 75, 76, 77, 78, 79, 80, 81, 82, 83, 84, 85% or more. Generally, segment 3 and segment 3C have 55-70% GC content. In one embodiment segment 3 and segment 3C have approximately 60-65% GC content.

Typically, where double stranded polynucleotide is being synthesised, additional nucleotide 1 and additional nucleotide 2 are complementary to each other.

Thus, in one embodiment additional nucleotide 1 is complementary to additional nucleotide 2.

In one embodiment the first and second ligation strands are ligated simultaneously.

Ligating as employed herein means covalent joining of two nucleotide strands by the formation of a phosphodiester bond. Typically, as employed herein ligation occurs by means of a ligase enzyme.

Steps b) to d) of the method may be repeated as many times as desired to grow/synthesise a polynucleotide strand of desired sequence and length.

The nucleotide synthesis method described herein can be applied on the microfluidic platform as disclosed in patent application No. GB201901273.0 filed on 30.01.2019, which is equipped with bespoke automated components that allow controlled flow of chemical reagents, biomolecules or enzymes through single or multiple channels and also possess selective temperature control capability.

Typically, the channel size is approximately 400 µm, channel height is approximately 800 µm and reaction chamber holds approximately 90 µl.

The microfluidic device could be made, for example, of the following components; polydimethylsiloxane, glass, polycarbonate, polyethylene terephthalate or any organic polymer that contains a high content of halogen atoms.

A complete automation cycle includes, for example, priming and flushing the flow cell with buffer. An example method may include the steps: Reaction one is initiated using reagent A which is flowed through the channel through the inlet port at a controlled velocity. Upon completion of enzymatic step, the reaction is terminated and remnant strands, enzymes, salts and other impurities are washed from the reaction chamber in the flowcell and collected in a reservoir through the outlet port. Flow cell wash is repeated three times. Reaction two is initiated using reagent B which is flowed through the channel through the inlet port. Flowcell washing and waste collection remains the same as mentioned above expect at the wash step which is repeated five times. Polynucleotide synthesis (i.e. N+1) is achieved by flowing reagent C together with the desired nucleotides through the inlet port. Flow is held at the synthesis spot and enzymatic activity is optimised at room temperature. Flow cell wash and removal of impurities is carried out as mentioned above. Synthesised polynucleotide strand is harvested using reagent D. Harvesting of synthesised strands is only carried out after numerous cycles to the desired length of N+X.

### Examples

### Experimental result using scheme 1

### Designed oligo sequence

| **Oligo Name** | **Nucleotide sequence** | **Length** | **Modifications** |
|---|---|---|---|
| FMST01 | /5- FAM/CGACGCATCG/X/NNNATTTAAATCAGCCTAGC CGCG/3Bio/ | 34bases | X =Inosine, bio = Biotin |
| | | | Fluorophore = FAM |
| CMFM01 | CGCGGCTAGGCTGATTTAAATC/Y/NNNGAT/3-FAM/ | 28bases | Y= Uracil |
| | | | Fluorophore = FAM |
| OLO strand | A/Y/NNNAGATTCTAGTCTCTCTATCTACTCCTAGCTC GATCTAT/3-FAM/ | 42bases | Y= Uracil |
| | | | Fluorophore = FAM |
| BDRSD strand | /5- FAM/ACGGATAGATCGAGCTAGGAGTAGATAGAGA GACTAGAATCTN/X/NNT | 46bases | X = Inosine |
| | | | Fluorophore = FAM |

| | | | |
|---|---|---|---|
| N.B: Highlighted (NNN) sequence is the conserved unique constant region (CUCM) | | | |

### Experimental Conditions

### Step 1: Polynucleotide immobilisation and dual cleavage (Figure 4 and 5).

### Materials

Starting polynucleotides used were designed in-house and synthesised by by a commercial provider (see table above for sequences). The polynucleotides were designed to a scale of 100uM and with a purification scale of standard desalting and HPLC. Dilution to desired concentration was carried out with elution buffer (EB) (QIAGEN) and stored appropriately for subsequent reaction. Quality and integrity of polynucleotides were determined using a NanoDrop one (Thermo Fisher Scientific). Equilibration was typically carried out with EB buffer. Blanking was initiated after gently lowering arm of the device. Measurement was carried out at A260/A280 and also A260/A230. Immobilisation of starting polynucleotide was carried out on Dynabeads^{®} M-270 Streptavidin. This had a high affinity of binding with our designed polynucleotide coupled with biotin (see table 1 for sequence). Streptavidin- biotin interaction is Kd=10-15. The streptavidin dynabeads used had a bead diameter of 2.8um and an iron content (Ferrites) of 14%. Surface functionality included carboxylic acid and of a very hydrophilic nature.

### Methods

Polynucleotide immobilization was carried in a 1X binding and washing buffer (B&W) which contained 10mM Tris-HCl (pH 7.5), 1mM EDTA and 2M NaCl. Coupling was carried out for 15mins after which strands were purified using a DynaMag-2 (Thermo Fisher). Successfully coupled polynucleotide strands were visualized using the UV transilluminator (UVP). Further washes were carried out with B&W buffer as required. Dual cleavage reaction was carried out using 10X buffer (30mM Tris-HCl (pH 7.5), 150nM NaCl, 1mM EDTA, 1mM DTT, 0.05% (w/v) Tween 20 and 50% (v/v) glycerol. 1X reaction buffer mixture was made and 100pmol of DNA strands was added into the same reaction vessel. 2µl of Uracil-DNA glycosylase (UDG) 200U Thermo Fisher was added into the same reaction vessel and mixed by gentle resuspension with a p100 Gilson pipette. Further mixing was carried out by a quick vortex. Cleavage was carried out at 37C for 1 hour. Cleaved remnant strands and other impurities were removed by applying the DynaMag-2 (Thermo Fisher). Unbound cleaved remnant strands were removed from the mixture using a p100 Gilson pipette. Three separate washes with B&W buffer was carried out to remove any residual contaminants. Subsequent abasic site removal was carried out either by enzymatic or chemical approach.

The second step of cleavage was carried out by adding 1X buffer (50mM Potassium acetate, 20mM Tris-acetate, 10mM Magnesium Acetate, 1mM DTT at pH 7.9.). The buffer was added to the biotinylated polynucleotide present in the reaction vessel and gently resuspended with a p100 Gilson pipette. 2µl of Endonuclease V 250U (NEB) cleavage enzyme was added into the same vessel and mixed by gentle resuspension with a p100 Gilson pipette. Further mixing was carried out by a quick vortex. Cleavage was carried out at 37C for 1 hour. The reaction was brought to a stop by heat inactivation at 70C for 10mins. Cleaved remnant strands and other impurities was removed by applying the DynaMag (Thermo Fisher). Unbound cleaved remnant strands were removed from the mixture using a p100 Gilson pipette. Six separate washes were carried out with B&W buffer to remove any contaminants and denatured enzymes. Bound biotinylated dual cleaved polynucleotide strand was detached from the streptavidin molecule by the addition of sterile de-ionized water and heating to 75C for 2mins. This reaction is reversible hence the streptavidin beads could be reused. Other methods for detachment of biotin from streptavidin include competitive binding elution by using free biotin molecules, altering the pH of the solution or altering the electromagnetic field.

Dual cleaved reaction success was checked for fluorescence using a UV transilluminator and also gel electrophoresis method using the X-cell sue lock module (Novex) and a 15% TBE-Urea gel 1.0mm x 10 well (Invitrogen). 3µl of DNA strands were loaded in the individual wells together with 5µl of 6X Urea and loading buffer (Novex). Samples were run in a 1X TBE buffer (Thermo Scientific). Electrophoresis was performed at the following conditions 300V, 90Amps for 35mins at room temperature. DNA visualization was carried with an iBright FL1000 imager (Thermo Fisher Scientific) at the appropriate fluorophore excitation and emission wavelengths. Downstream DNA analysis was carried out using the iBright analysis software and web based Thermo Fisher cloud platform.

Step 2: Ligation of 4 polynucleotide strands on immobilized DNA (Figure 7). Two of the strands must contain the variable region and the CUCM and all strands must contain the compactible 3' terminus-hydroxyl group and 5' terminus-phosphate group.

### Materials

DNA strands purified from step 1 above was quantified using NanoDrop one (Thermo Scientific). Equilibration was typically carried out with EB buffer. Blanking was initiated after gently lowering arm of the device. Measurement was carried out at A260/A280 and also A260/A230. Immobilisation of polynucleotide strands was carried out on Dynabeads^{®} M-270 Streptavidin. The second set of polynucleotides (see table 1 for sequence) to be ligated was added into the reaction vessel together with the cleaved immobilized polynucleotide from step 1.

### Methods

Ligation was carried out at ambient room temperature under these conditions. 20µl of 1x Blunt end ligation master mix (T4 ligase) was added to 5µl of the cleaved re-bound polynucleotide and 5µl of the new set of polynucleotides to be ligated. The mixture was gently resuspended using a p100 pipette and further mixed by briefly vortexing. Ligation was carried out for 1hour. Newly ligated product was further treated with Lambda Endonuclease to eliminate any unligated 5' terminus phosphate acceptor strands. Ligation reaction was purified with B&W washing step. Bound newly ligated polynucleotides were detached from the streptavidin molecules by the addition of sterile distilled water and heating to 75C for 2mins. The success of ligation was checked for fluorescence using a UV transilluminator and also gel electrophoresis method using the X-cell sure lock module (Novex) and a 15% TBE-Urea gel 1.0mm x 10 well (Invitrogen). 3µl of DNA strands were loaded in the individual wells together with 5µl of 6X Urea and loading buffer (Novex). Samples were run in a 1X TBE buffer (Thermo Scientific). Electrophoresis was performed at the following conditions 300V, 90Amps for 35mins at room temperature. DNA visualization was carried with an iBright FL1000 imager (Thermo Fisher scientific) at the appropriate fluorophore excitation and emission wavelengths. Downstream DNA analysis was carried out using the iBright Analysis Software and web based Thermo Fisher cloud platform.

### Hairpin method - scheme 2

### Step 1: Polynucleotide immobilisation and single hairpin loop merger, dual cleavage (Figure 10).

Starting polynucleotides used were designed in-house and synthesised by a commercial oligonucleotide company (see table below for sequences). Single hairpin contained an internal fluorophore withing a deoxythymidine rich backbone loop for monitoring enzymatic reactions. The polynucleotides were designed to a scale of 100uM and with a purification scale of standard desalting and HPLC. Dilution to desired concentration was carried out with elution buffer (EB) (QIAGEN) and stored appropriately for subsequent reaction. Quality and integrity of polynucleotides were determined using a NanoDrop one (Thermo Scientific). Equilibration was typically carried out with EB buffer. Blanking was initiated after gently lowering arm of the device. Measurement was carried out at A260/A280 and also A260/A230. Immobilisation of starting polynucleotide was carried out on Dynabeads^{®} M-270 Streptavidin. This had a high affinity of binding with our designed polynucleotide coupled with biotin (see table 2 for sequence). Streptavidin- biotin interaction is Kd=10-15. The streptavidin dynabeads used had a bead diameter of 2.8um and an Iron content (Ferrites) of 14%. Surface functionality included carboxylic acid and of a very hydrophilic nature.

Polynucleotide immobilization was carried in a 1X binding and washing buffer (B&W) which contained 10mM Tris-HCl (pH 7.5), 1mM EDTA and 2M NaCl. Coupling was carried out for 15mins after which strands were purified using a DynaMag-2 (Thermofisher). Successfully coupled polynucleotide strands were visualized using the UV transilluminator (UVP). Further washes were carried out with B&W buffer as required. Dual cleavage reaction was carried out using 10X buffer (30mM Tris-HCl (pH 7.5), 150nM NaCl, 1mM EDTA, 1mM DTT, 0.05% (w/v) Tween 20 and 50% (v/v) glycerol. 1X reaction buffer mixture was made and 100pmol of DNA strands was added into the same reaction vessel. 2µl of Uracil-DNA glycoslase (UDG) 200U Thermofisher was added into the same reaction vessel and mixed by gentle resuspension with a p100 gilson pipette. Further mixing was carried out by a quick vortex. Cleavage was carried out at 37C for 1 hour. Cleaved remnant strands and other impurities were removed by applying the DynaMag-2 (Thermofisher). Unbound cleaved remnant strands were removed from the mixture using a p100 gilson pipette. Three separate washes with B&W buffer was carried out to remove any residual contaminants. Subsequent abasic site removal was carried out either by enzymatic or chemical approach.

The second step of cleavage was carried out by adding 1X buffer (50mM Potassium acetate, 20mM Tris-acetate, 10mM Magnesium Acetate, 1mM DTT at pH 7.9.). The buffer was added to the biotylated polynucleotide present in the reaction vessel and gently resuspended with a p100 gilson pipette. 2µl of Endonuclease V 250U (NEB) cleavage enzyme was added into the same vessel and mixed by gentle resuspension with a p100 gilson pipette. Further mixing was carried out by a quick vortex. Cleavage was carried out at 37C for 1 hour. The reaction was brought to a stop by heat inactivation at 70C for 10mins. Cleaved remnant strands and other impurities was removed by applying the DynaMag (Thermofisher). Unbound cleaved remnant strands were removed from the mixture using a p100 gilson pipette. Six separate washes were carried out with B&W buffer to remove any contaminants and denatured enzymes. Bound biotylated dual cleaved polynucleotide strand was detached from the streptavidine molecule by the addition of sterile de-ionized water and heating to 75C for 2mins. This reaction is reversible hence the streptavidin beads could be reused. Other methods for detachment of biotin from streptavidin include competitive binding elution by using free biotin molecules, altering the pH of the solution, or altering the electromagnetic field.

Dual cleaved reaction success was checked for fluorescence using a UV transillminator and also gel electrophoresis method using the X-cell sue lock module (Novex) and a 15% TBE-Urea gel 1.0mm x 10 well (Invitrogen). 3µl of DNA strands were loaded in the individual wells together with 5µl of 6X Urea and loading buffer (Novex). Samples were ran in a 1X TBE buffer (Thermo Scientific). Electrophoresis was performed at the following conditions 300V, 90Amps for 35mins at room temperature. DNA visualization was carried with an iBright FL1000 imager (Thermofisher scientific) at the appropriate fluorophore excitation and emission wavelengths. Downstream DNA analysis was carried out using the iBright analysis software and web based ThermoFisher cloud platform.

### Step 2- ligations of hairpin looped polynucleotide strands on immobilised DNA

DNA strands purified from step 1 above was quantified using NanoDrop one (Themo Scientific). Equilibration was typically carried out with EB buffer. Blanking was initiated after gently lowering arm of the device. Measurement was carried out at A260/A280 and also A260/A230. Immobilisation of polynucleotide strands was carried out on Dynabeads^{®} M-270 Streptavidin. The second set of hairpin looped polynucleotides (see table 1 for sequence) to be ligated was added into the reaction vessel together with the cleaved immobilized polynucleotide from step 1.

Ligation was carried out at ambient room temperature under these conditions. 20µl of 1x Blunt end ligation master mix (T4 ligase) was added to 5µl of the cleaved re-bound polynucleotide and 5µl of the new set of single hairpin polynucleotides to be ligated. The mixture was gently esuspended using a p100 pipette and further mixed by briefly vortexing. Ligation was carried out for 1hour. Newly ligated product was further treated with Lambda Endonuclease to eliminate any unligated 5' terminus phosphate acceptor strands. Ligation reaction was purified with B&W washing step. Bound newly ligated polynucleotides were detached from the streptavidine molecules by the addition of sterile distilled water and heating to 75C for 2mins. Multiple successive synthesis cycle and incorporation of all four natural nucleotides (A, T, G, C) was carried out by repeating steps 1 and 2 above. One complete synthesis cycle was achieved within 30mins upon optimization using in-house formulated reaction buffer which remains a trade secret. Experimental data can be seen below. The success of ligation was checked for fluorescence using a UV transillminator and also gel electrophoresis method using the X-cell sure lock module (Novex) and a 15% TBE-Urea gel 1.0mm x 10 well (Invitrogen). 3µl of DNA strands were loaded in the individual wells together with 5µl of 6X Urea and loading buffer (Novex). Samples were ran in a 1X TBE buffer (Thermo Scientific). Electrophoresis was performed at the following conditions 300V, 90Amps for 35mins at room temperature. DNA visualization was carried with an iBright FL1000 imager (Thermofisher scientific) at the appropriate fluorophore excitation and emission wavelengths. Downstream DNA analysis was carried out using the iBright Analysis Software and web based Thermo Fisher cloud platform.

**Table 2**

| Oligo Name | Nucleotide Sequence | Lengt h | Modification s |
|---|---|---|---|
| FMST01 | /5- FAM/CGACGCATCG/X/NNNATTTAAATCAGCCTAGCCGCG/3Bio/ | 34 bases | X =Glycoslase site 1, Bio = Biotin |
| | | | Fluorophore = FAM |
| CMFM0 1 | CGCGGCTAGGCTGATTTAAATC/Y/N N NGAT/3-FAM/ | 28 bases | Y= Glycoslase site 2 |
| | | | Fluorophore = FAM |
| SVHPV1 | C/Y/NNNTAGGTCCTATACAGATACCTAGATTT/iFluorT/TTTTCTAG GT ATCTGTATAGGACCTAN/X/NNG | 60 bases | Y= Glycoslase site 2 |
| | | | X = Glycoslase site 1 |
| | | | Fluorophore = Internal FAM |

## Claims

1. A method of synthesising single or double-stranded polynucleotide comprising the steps:
a) providing a polynucleotide starter strand comprising, in sequence from 5' to 3', a nucleotide strand selected from the group consisting:
(i) 5' blocker - 5' end - segment 1- CUCM 1 - segment 2-3' end wherein the 3' end is either immobilised or blocked, and,
optionally, a hybridised complementary strand comprising, in sequence from 5' to 3' 5' end - segment 2C - CUCM2 - segment 1C - 3'end - 3' blocker wherein the 5' end is either immobilised or blocked,
(ii) 5' blocker-5' end - segment 1- CUCM1- segment 2 - HP1-segment 2C - CUCM2 - segment 1C - 3'end - 3' blocker wherein HP1 is optionally immobilised, and
(iii) segment 1 - CUCM1 - segment 2 - HP1 - segment 2C - CUCM2 - segment 1C - HP2 wherein segment 1 is linked to HP 2 to form a double hairpin structure, wherein either HP1 or HP2 may optionally have a HP fluorophore attached and wherein, optionally, the other of HP1 and HP2 is immobilised,
b) digesting CUCM1 with glycosylase enzyme 1 or CUCM2 with a glycosylase enzyme 2 and washing the starter strand to remove the remnant of the digest,
c) where the method is employed to synthesise double stranded polynucleotide, digesting the other of CUCM1 with a first glycosylase enzyme 1 or CUCM2 with glycosylase enzyme 2 and washing to remove the remnant of the digest,
d) ligating a first ligation strand comprising: 5' blocker - 5' end - segment 3 - CUCM3 - additional nucleotide 1-3' end, and/or a second ligation strand comprising: 5' end - additional nucleotide 2 - CUCM4 - segment 3C - 3' end - 3' blocker to the starter strand, and
e) repeating steps b) to d) as many times as required to provide the desired synthetic polynucleotide.

2. The method according to claim 1 wherein 5' blocker and the 3' blocker are each independently selected from the group consisting: a spacer or a fluorophore.

3. The method according to claim 2 wherein the spacer is selected from the group consisting of: spacer C3, spacer C6, spacer C9, spacer C12 and spacer C18.

4. The method according to claim 2 wherein the fluorophore is selected from the group consisting of: TAMRA, Cy3, Cy5, rhodamine red-X, rhodol green, Texas red-X, Oregon green 488/500/514, VIC, Alexa Fluor 488/532/542/555/594/647/750 or FAM

5. The method according to any preceding claim wherein CUCM1 and CUCM2 each independently has the sequence N(x)ZN(y) wherein N is a nucleotide, x and y are each independently a number in the range 0 to 8 and Z is a glycosylase recognition site.

6. The method according to claim 5 wherein the sum of x+y is less than 8.

7. The method according to claim 6 wherein the sum of x+y is 4.

8. The method according to any preceding claim wherein segment 1, segment 1C, segment 2 and segment 2C are each 20-80 nucleotides long.

9. The method according to claim 8 wherein segment 1, segment 1C, segment 2 and segment 2C are each at least 25 nucleotides long and/or at most 80 nucleotides long.

10. The method according to any one of claims 8 or 9 wherein segment 1, segment 1C, segment 2 and segment 2C are each approximately 40 nucleotides long.

11. The method according to any preceding claim wherein the product of step c) is a blunt ended double stranded nucleotide.

12. The method according to any preceding claim wherein steps b) and c) are performed simultaneously.

13. The method according to any preceding claim wherein the first and second ligation strands are ligated simultaneously.

14. The method of preceding claim wherein the first and second ligation strand are joined by a hairpin loop.

15. A kit of parts comprising:
a) a polynucleotide starter strand comprising, in sequence from 5' to 3', a nucleotide strand selected from the group consisting:
(i) 5' blocker - 5' end - segment 1 - CUCM1 - segment 2-3' end wherein the 3' end is either immobilised or blocked, and,
optionally, a hybridised complementary strand comprising, in sequence from 5' to 3' 5' end - segment 2C - CUCM2 - segment 1C - 3' end - 3' blocker wherein the 5' end is either immobilised or blocked,
(ii) 5' blocker-5' end - segment 1- CUCM1- segment 2- HP 1- segment 2C-CUCM2-segment 1C - 3' end - 3' blocker wherein HP1 is optionally immobilised, and
(iii) segment 1-CUCM1-segment 2 - HP 1 - segment 2C - CUCM2 - segment 1C- HP 2 wherein segment 1 is linked to HP 2 to form a double hairpin structure, wherein either HP1 or HP2 optionally has a HP fluorophore attached and wherein, optionally, the other of HP1 and HP2 is immobilised,
(iv) optionally one or more glycosylase enzymes,
(v) optionally a ligase enzyme, and
(vi) a first ligation strand comprising: 5' blocker-5' end - segment 1- CUCM1- additional nucleotide 1-3' end, and/or a second ligation strand comprising: 5' end - additional nucleotide 2 - segment 2C - CUCM2 - segment 1C - 3' end - 3' blocker, optionally wherein the first ligation strand and the second ligation strand are joined by a hairpin loop.

## Patentansprüche

1. Verfahren zum Synthetisieren von einzel- oder doppelsträngigem Polynukleotid, umfassend die folgenden Schritte:
a) Bereitstellen eines Polynukleotid-Starterstrangs, der in Reihenfolge von 5' zu 3' einen Nukleotidstrang umfasst, ausgewählt aus der Gruppe bestehend aus:
(i) 5'-Blocker - 5'-Ende - Segment 1 - CUCM1 - Segment 2 - 3'-Ende, wobei das 3'-Ende entweder immobilisiert oder blockiert ist, und
optional einem hybridisierten komplementären Strang, umfassend, in Reihenfolge von 5'-bis 3' 5'-Ende - Segment 2C - CUCM2 - Segment 1C - 3'-Ende - 3'-Blocker, wobei das 5'-Ende entweder immobilisiert oder blockiert ist,
(ii) 5'-Blocker - 5'-Ende - Segment 1 - CUCM1 - Segment 2 - HP1 - Segment 2C - CUCM2 - Segment 1C - 3'-Ende - 3'-Blocker, wobei HP1 optional immobilisiert ist, und
(iii) Segment 1 - CUCM1 - Segment 2 - HP1 - Segment 2C - CUCM2 - Segment 1C - HP2, wobei Segment 1 mit HP 2 verbunden ist, um eine doppelte Haarnadelstruktur zu bilden, wobei entweder HP1 oder HP2 optional ein angehängtes HP-Fluorophor aufweisen kann und wobei optional das andere von HP1 und HP2 immobilisiert ist,
b) Verdauen von CUCM1 mit Glycosylase-Enzym 1 oder CUCM2 mit einem Glycosylase-Enzym 2 und Waschen des Starterstrangs, um den Rest des Verdaus zu entfernen,
c) wenn das Verfahren eingesetzt wird, um doppelsträngiges Polynukleotid zu synthetisieren, Verdauen des anderen von CUCM1 mit einem ersten Glycosylase-Enzym 1 oder CUCM2 mit Glycosylase-Enzym 2 und Waschen, um den Rest des Verdaus zu entfernen,
d) Ligieren eines ersten Ligationsstrangs, umfassend: 5'-Blocker - 5'-Ende - Segment 3 - CUCM3 - zusätzliches Nukleotid 1 - 3'-Ende, und/oder eines zweiten Ligationsstrangs, umfassend: 5'-Ende - zusätzliches Nukleotid 2 - CUCM4 - Segment 3C - 3'-Ende - 3'-Blocker zu dem Starterstrang, und
e) Wiederholen der Schritte b) bis d) so oft wie nötig, um das gewünschte synthetische Polynukleotid bereitzustellen.

2. Verfahren nach Anspruch 1, wobei 5'-Blocker und der 3'-Blocker jeweils unabhängig ausgewählt sind aus der Gruppe bestehend aus: einem Spacer oder einem Fluorophor.

3. Verfahren nach Anspruch 2, wobei der Spacer ausgewählt ist aus der Gruppe bestehend aus: Spacer C3, Spacer C6, Spacer C9, Spacer C12 und Spacer C18.

4. Verfahren nach Anspruch 2, wobei das Fluorophor ausgewählt ist aus der Gruppe bestehend aus TAMRA, Cy3, Cy5, Rhodaminrot-X, Rhodolgrün, Texasrot-X, Oregongrün 488/500/514, VIC, Alexa Fluor 488/532/542/555/594/647/750 oder FAM.

5. Verfahren nach einem vorhergehenden Anspruch, wobei CUCM1 und CUCM2 jeweils unabhängig die Sequenz N(x)ZN(y) aufweisen, wobei N ein Nukleotid ist, x und y jeweils unabhängig eine Zahl in dem Bereich 0 bis 8 sind und Z eine Glycosylase-Erkennungsstelle ist.

6. Verfahren nach Anspruch 5, wobei die Summe von x+y weniger als 8 ist.

7. Verfahren nach Anspruch 6, wobei die Summe von x+y 4 ist.

8. Verfahren nach einem vorhergehenden Anspruch, wobei Segment 1, Segment 1C, Segment 2 und Segment 2C jeweils 20-80 Nukleotide lang sind.

9. Verfahren nach Anspruch 8, wobei Segment 1, Segment 1C, Segment 2 und Segment 2C jeweils zumindest 25 Nukleotide lang und/oder höchstens 80 Nukleotide lang sind.

10. Verfahren nach einem der Ansprüche 8 oder 9, wobei Segment 1, Segment 1C, Segment 2 und Segment 2C jeweils ungefähr 40 Nukleotide lang sind.

11. Verfahren nach einem vorhergehenden Anspruch, wobei das Produkt von Schritt c) ein doppelsträngiges Nukleotid mit stumpfen Enden ist.

12. Verfahren nach einem vorhergehenden Anspruch, wobei Schritt b) und c) gleichzeitig durchgeführt werden.

13. Verfahren nach einem vorhergehenden Anspruch, wobei der erste und der zweite Ligationsstrang gleichzeitig ligiert werden.

14. Verfahren nach vorhergehendem Anspruch, wobei der erste und der zweite Ligationsstrang durch eine Haarnadelschleife verknüpft sind.

15. Kit von Teilen, umfassend:
a) einen Polynukleotid-Starterstrang, der in Reihenfolge von 5' bis 3' einen Nukleotidstrang umfasst, der ausgewählt ist aus der Gruppe bestehend aus:
(i) 5'-Blocker - 5'-Ende - Segment 1 - CUCM1 - Segment 2 - 3'-Ende, wobei das 3'-Ende entweder immobilisiert oder blockiert ist, und
optional einem hybridisierten komplementären Strang, umfassend, in Reihenfolge von 5'- bis 3' 5'-Ende - Segment 2C - CUCM2 - Segment 1C - 3'-Ende - 3'-Blocker, wobei das 5'-Ende entweder immobilisiert oder blockiert ist,
(ii) 5'-Blocker - 5'-Ende - Segment 1 - CUCM1 - Segment 2 - HP 1 - Segment 2C - CUCM2 - Segment 1C - 3'-Ende - 3'-Blocker, wobei HP 1 optional immobilisiert ist, und
(iii) Segment 1 - CUCM1 - Segment 2 - HP 1 - Segment 2C - CUCM2 - Segment 1C - HP 2, wobei Segment 1 mit HP 2 verbunden ist, um eine doppelte Haarnadelstruktur zu bilden, wobei entweder HP1 oder HP2 optional ein angehängtes HP-Fluorophor aufweist und wobei optional das andere von HP1 und HP2 immobilisiert ist,
(iv) optional einem oder mehreren Glycosylase-Enzymen,
(v) optional einem Ligaseenzym, und
(vi) einem ersten Ligationsstrang, umfassend: 5'-Blocker - 5'-Ende - Segment 1 - CUCM1 - zusätzliches Nukleotid 1 - 3'-Ende, und/oder einen zweiten Ligationsstrang, umfassend: 5'-Ende - zusätzliches Nukleotid 2 - Segment 2C - CUCM2 - Segment 1C - 3'-Ende - 3'-Blocker, wobei optional der erste Ligationsstrang und der zweite Ligationsstrang durch eine Haarnadelschleife verknüpft sind.

## Revendications

1. Procédé de synthèse de polynucléotide simple ou double brin comprenant les étapes de :
a) fourniture d'un brin d'initiation polynucléotidique comprenant, en séquence de 5' à 3', un brin nucléotidique choisi dans le groupe constitué de :
(i) bloqueur 5' - extrémité 5' - segment 1 - CUCM1 - segment 2 - extrémité 3' où l'extrémité 3' est soit immobilisée, soit bloquée, et,
facultativement, un brin complémentaire hybridé comprenant, en séquence de 5' à 3', extrémité 5' - segment 2C - CUCM2 - segment 1C - extrémité 3' - bloqueur 3' où l'extrémité 5' est soit immobilisée, soit bloquée,
(ii) bloqueur 5' - extrémité 5' - segment 1 - CUCM1 -segment 2 - HP1 - segment 2C - CUCM2 - segment 1C - extrémité 3' - bloqueur 3' où HP1 est facultativement immobilisé, et
(iii) segment 1 - CUCM1 - segment 2 - HP1 - segment 2C - CUCM2 - segment 1C - HP2 où le segment 1 est relié à HP 2 pour former une structure en épingle à cheveux double, où HP1 ou HP2 peuvent éventuellement avoir un fluorophore HP attaché et où, éventuellement, l'autre de HP1 et HP2 est immobilisé,
b) digestion de CUCM1 avec l'enzyme glycosylase 1 ou de CUCM2 avec une enzyme glycosylase 2 et nettoyage du brin d'initiation pour éliminer le reste du produit de digestion,
c) où le procédé est utilisé pour synthétiser un polynucléotide double brin, par digestion de l'autre de CUCM1 avec une première enzyme glycosylase 1 ou de CUCM2 avec l'enzyme glycosylase 2 et nettoyage pour éliminer le reste du produit de digestion,
d) ligature d'un premier brin de ligature comprenant : bloqueur 5' - extrémité 5' - segment 3 - CUCM3 - nucléotide supplémentaire 1 - extrémité 3', et/ou un deuxième brin de ligature comprenant : extrémité 5' - nucléotide supplémentaire 2 - CUCM4 - segment 3C - extrémité 3' - bloqueur 3' au brin d'initiation, et
e) répétition des étapes b) à d) autant de fois que nécessaire pour fournir le polynucléotide synthétique souhaité.

2. Procédé selon la revendication 1, dans lequel le bloqueur 5' et le bloqueur 3' sont chacun indépendamment sélectionnés dans le groupe constitué de : un espaceur ou un fluorophore.

3. Procédé selon la revendication 2, dans lequel l'espaceur est choisi dans le groupe constitué de : l'espaceur C3, l'espaceur C6, l'espaceur C9, l'espaceur C12 et l'espaceur C18.

4. Procédé selon la revendication 2, dans lequel le fluorophore est choisi dans le groupe constitué par : TAMRA, Cy3, Cy5, rhodamine rouge-X, vert rhodol, Texas rouge-X, vert Oregon 488/500/514, VIC, Alexa Fluor 488/532/542/555/594/647/750 ou FAM.

5. Procédé selon l'une quelconque des revendications précédentes, dans lequel CUCM1 et CUCM2 ont chacun indépendamment la séquence N(x)ZN(y) dans laquelle N est un nucléotide, x et y sont chacun indépendamment un nombre compris entre 0 et 8 et Z est un site de reconnaissance par glycosylase.

6. Procédé selon la revendication 5, dans lequel la somme de x+y est inférieure à 8.

7. Procédé selon la revendication 6, dans lequel la somme de x+y est 4.

8. Procédé selon l'une quelconque des revendications précédentes, dans lequel le segment 1, le segment 1C, le segment 2 et le segment 2C ont chacun une longueur de 20 à 80 nucléotides.

9. Procédé selon la revendication 8, dans lequel le segment 1, le segment 1C, le segment 2 et le segment 2C ont chacun une longueur d'au moins 25 nucléotides et/ou une longueur d'au plus 80 nucléotides.

10. Procédé selon l'une quelconque des revendications 8 ou 9, dans lequel le segment 1, le segment 1C, le segment 2 et le segment 2C ont chacun une longueur d'environ 40 nucléotides.

11. Procédé selon l'une quelconque des revendications précédentes, dans lequel le produit de l'étape c) est un nucléotide double brin à extrémités franches.

12. Procédé selon l'une quelconque des revendications précédentes, dans lequel les étapes b) et c) sont effectuées simultanément.

13. Procédé selon l'une quelconque des revendications précédentes, dans lequel les premier et deuxième brins de ligature sont ligaturés simultanément.

14. Procédé selon la revendication précédente, dans lequel les premier et deuxième brins de ligature sont réunis par une boucle en épingle à cheveux.

15. Kit de pièces comprenant :
a) un brin d'initiation polynucléotidique comprenant, en séquence de 5' à 3', un brin nucléotidique choisi dans le groupe constitué par :
(i) bloqueur 5' - extrémité 5' - segment 1 - CUCM1 - segment 2 - extrémité 3' où l'extrémité 3' est soit immobilisée, soit bloquée, et,
éventuellement, un brin complémentaire hybridé comprenant, en séquence de 5' à 3', extrémité 5' - segment 2C - CUCM2 - segment 1C - extrémité 3' - bloqueur 3' où l'extrémité 5' est soit immobilisée soit bloquée,
(ii) bloqueur 5' - extrémité 5' - segment 1 - CUCM1- segment 2 - HP1 - segment 2C - CUCM2 - segment 1C - extrémité 3' - bloqueur 3' où HP1 est facultativement immobilisé, et
(iii) segment 1 - CUCM1 - segment 2 - HP 1 - segment 2C - CUCM2 - segment 1C - HP 2 où le segment 1 est relié à HP 2 pour former une structure en épingle à cheveux double, où HP1 ou HP2 a éventuellement un fluorophore HP attaché et où, éventuellement, l'autre de HP1 et HP2 est immobilisé,
(iv) éventuellement une ou plusieurs enzymes glycosylases,
(v) éventuellement une enzyme ligase, et
(vi) un premier brin de ligature comprenant : bloqueur 5' - extrémité 5' - segment 1 - CUCM1 - nucléotide supplémentaire 1 - extrémité 3', et/ou un deuxième brin de ligature comprenant : extrémité 5' - nucléotide supplémentaire 2 - segment 2C - CUCM2 - segment 1C - extrémité 3' - bloqueur 3', éventuellement où le premier brin de ligature et le deuxième brin de ligature sont reliés par une boucle en épingle à cheveux.
